# EUROPEAN PATENT APPLICATION

(11) **EP 1 308 510 A1**
(43) Date of publication of application: **07.05.2003**
(21) Application number: 01955624.0
(22) Date of filing: 09.08.2001
(51) Int. Cl.: C12N 15/11, C12N 1/15, C12N 1/19, C12N 1/21, C12N 5/00, C12Q 1/02, C12Q 1/68

(54) **GLYCERYLALDEHYDE-3-PHOSPHATE DEHYDROGENASE PROMOTER INDUCING CELL DEATH**

(30) Priority: 10.08.2000 JP 2000242014
(71) Applicant: ONO PHARMACEUTICAL CO., LTD., Osaka-shi, Osaka 541-8526 (JP)
(72) Inventor: ISHITANI, Ryoichi, Shiki-shi Saitama 353-0007 (JP)
(74) Representative: Henkel, Feiler, Hänzel
(86) International application number: JP0106858
(87) International publication number: WO02014509

(57) **Abstract**

Glyceraldehyde-3-phosphate dehydrogenase (GAPDH) promoter DNA which induces cell death, and a screening method for an apoptosis-suppressing agent using it.

According to this invention, an apoptosis-suppressing agent can be screened by measuring the promoter activity of the GAPDH gene which discovers specifically the GAPDH protein which guides cell death in cell death process using the host cell in which the transformation was carried out by the vector incorporating the GAPDH promoter DNA.

## Description

### Technical Field

This invention relates to glyceraldehyde-3-phosphate dehydrogenase (it may abbreviate to GAPDH) promoter DNA from rats which induces cell death (apoptosis), and uses thereof.

### Background Technology

Glyceraldehyde-3-phosphate dehydrogenase (GAPDH) is known as one of the main enzymes in the glycolysis system which carries out phosphorylation of glyceraldehyde-3-phosphate with nicotinamide adenine dinucleotide or nicotinamide adenine dinucleotide phosphates as a coenzyme, and G3PD is outlined.

It has found out by these inventors that GAPDH is deeply concerned with the apoptosis advocated by Kerr, Wyllie et al (programmed cell death, Brit. J. Cancer, 26, and 239 (1972)).

The present inventors reported that apoptosis arises suddenly after two weeks or more progress from culture starts, in continuation culture of rat cerebellum granule cells (CGCs) is carried out under KCl (25 mM) existence without exchange to new culture media, or without supplement of glucose (Japanese Patent Publication No. 8-92127). Since age-induced apoptosis of CGCs is suppressed by N-methyl-D-aspartate (NMDA) receptor antagonists or anti-oxidizers, it is thought that such apoptosis is based on an excess stimulation of the NMDA receptor by the glutamic acid which secreted from CGCs, or the oxygen radicals formed after that stimulation.

Furthermore, it has been also clear that over-expression of GAPDH is involving closely at this age-induced apoptosis of CGCs.

In decreasing in GAPDH mRNA expression by using GAPDH anti-sense oligo nucleotides, over-expression of GAPDH protein was suppressed and age-induced nerve cell apoptosis failed.

Therefore, apoptosis can be decreasing in over-expression of GAPDH mRNA or GAPDH protein.

Over-expression of GAPDH protein caused nuclear translocation of this protein, and increased apoptosis notably, not only in nerve cells but in cell lines from peripheral tissues (NeuroReport, 10, 2029-2033(1999), Mol. Pharmacol., 53, 701-707(1998)).

Moreover, it's known that GAPDH associated with the gene products (especially triplet repeat protein) which is the pathogenic proteins in other neuro-degenerative disorders (Burke et al., 1996; Koshy et al., 1996) .

Such gene product meant for example, Huntingtin of Huntington disease (Nat. Med., 2, and 347-350 (1996)), atrophin of Dentatorubropallidoluysian atrophy (DRPLA) (Hum. Mol. Genet., 5, 1311-1318 (1996)), ataxin of hereditary spino-cerebellar ataxia (SCA-1), ataxin-3 of the Machado Joseph disease, and androgen receptor of spinal and bulbar muscular atrophy (SBMA) etc.

Therefore, it is thought that GAPDH has played a certain role in development and/or onset of these diseases.

On the other hand, GAPDH gene promoter DNA is located in the upstream region of the coding domain for the protein (GAPDH), and is a regulatory region to start an expression of the gene. Promoter specifies the start of transcription of the gene to mRNA by interacting with the basic transcription complex that consists of RNA polymerase.

The GAPDH Promoter DNA from yeast has been known (GenBank registration No. A15895).

However, there was no report of identification of promoter DNA in the past about ones from other species, especially higher animals. Furthermore, about the promoter activated during cell death process, it is not known at all.

### Disclosure of the Invention

This invention relates to isolated (pro-apoptotic) GAPDH promoter from rat which induces cell death and a screening method of an apoptosis-suppressing agent using this GAPDH promoter.

Namely, the present invention relates to:
(1) An isolated glyceraldehyde-3-phosphate dehydrogenase (GAPDH) promoter DNA,
(2) A vector introduced in GAPDH promoter DNA,
(3) Host cells transfected with vector containing with GAPDH promoter DNA,
(4) A screening method of an apoptosis-suppressing agent using rat GAPDH promoter DNA,
(5) A screening method of an apoptosis-suppressing agent using vector containing with rat GAPDH promoter DNA,
(6) A screening method of apoptosis-suppressing agent using host cells transformed by vector containing with rat GAPDH promoter DNA.

### Detailed Description of Invention

The rat GAPDH promoter DNA concerning this invention has the sequence shown in the SEQ ID NOs: 1, 3, and 5 and the SEQ ID NO: 6.

This invention also contains complementary sequence of ones shown in the SEQ ID NOs: 1, 3, 5, and 6.

The complementary DNAs have an inhibitory activity suppresse promoter DNA powerfully as an anti-sense chain.

Furthermore, this invention contains the vector composed of above DNA. As a vector used by this invention, the vector from a virus or a phage origin is mentioned, for example, pGL3 (Promega) can be mentioned. It is desirable that the index gene or marker one for screening is included in the vector said here.

Enzyme genes related to luminescence (Firefly luciferase etc.) can be used as a marker gene.

This invention contains the host cells transformed by the above-mentioned. As the host cells used by this invention, which are animal cell lines such as COS cells and PC12 cells suitable for transfection of genes are desirable, and rat cerebellum granule cells (CGCs) which produces apoptosis physiologically are especially desirable.

GAPDH is participating in apoptosis, as stated previously. GAPDH promoter promotes expression of GAPDH protein. Therefore, it can use for the medical treatment of the disease considered that over-expression of GAPDH is involving, especially the neuro-degenerative disorders characterized by lack of nerve cells.

By the screening method of this invention, the compounds, which suppress GAPDH promoter activity, can be screened.

### Preparation of promoter in this invention

It is prepared by the method below the glyceraldehyde-3-phosphate dehydrogenase (GAPDH) promoter of this invention.

Namely, it can acquire:
(1) By using rat genome DNA library,
(2) PCR is performed with complementary primer to the upstream region of the translation domain and complementary primer to adapter added to the library DNA,
(3) By choosing the DNA fragment by which unique amplification was carried out,
(4) By inserting the obtained DNA into a vector,
(5) By transforming host cells with the vector, and cultivating them,
(6) Colony hybridization is performed with rat GAPDH fragment as a probe,
(7) By extracting and purifying a plasmid DNA from positive colony.

### GAPDH promoter DNA

The GAPDH (pro-apoptotic) promoter DNA that induces rat cell death of this invention is the new sequence isolated for the first time from the higher animal. Moreover, when compared with the promoter DNA of the yeast GAPDH already known (GenBank registration number A15895), their sequence homology was less than 50%.

Downstream from nucleic acid sequence number 960 of SEQ ID NO: 1 (p104) and downstream from nucleic acid sequence number 2401 of SEQ ID NO: 3 has very high homology. However, most homology of an upstream from them is not seen.

### Screening Method for Apoptosis Suppresser

The screening method of an apoptosis-suppressing agent by using GAPDH promoter, the compound that can suppress expression of the pro-apoptotic GAPDH protein that induces cell death can be chosen. The compound, which suppresses promoter activation of a GAPDH gene especially, can be easily found out as an "apoptosis-suppressing agent."

More concretely, screening for apoptosis-suppressing agent may be carried out:
(1) By inserting rat GAPDH promoter into vector,
(2) By transforming host cells with the vector,
(3) By adding a tested compound and
(4) By measuring survival ratio of the host cells.

The rat GAPDH promoters DNA meant here are the sequences shown in SEQ ID NOs: 1, 3, 5, and 6, and sequences preferably shown in SEQ ID NO: 5 or 6.

Although the SEQ ID NOs: 1 and 3 contain the protein translation portion of rat GAPDH in a 3'-end in part, they can be used for screening of this invention.

The SEQ ID NOs: 2 and 4 show the translated protein of each 3'-end of the sequence.

A vector should just be a reporter vector currently used widely, preferable one is p104-pGL3-enhanser vector.

Host cells for screening may be ones can start apoptosis on physiological conditions, besides animal cell line, and preferable one is CGCs.

Measurement is performed using promotion of the expression of the protein encoded by the downstream gene when the promoter is activated, in the progress of apoptotic process of the host cells transfected with GAPDH promoter-containing vector.

It is desirable to use the host cell in which the transformation was carried out as control by the vector that has not inserted the GAPDH promoter.

In the case of measurement, promoter activity can be measured by the activity of some inserted indices, such as luciferase, as the downstream gene of the GAPDH promoter.

Moreover, when a GAPDH gene is directly inserted downstream of the promoter, it is good also as an index of suppression by counting the number of survival cells.

Tested compound may add just before or simultaneously of the addition of cell death induction agent. Timing of the addition of the compound may change if it does not reinforce the cell obstacle by DNA transfection.

Addition of cytosine arabinoside (AraC) and age-induced apoptosis can also be used as conditions for cell death induction.

Moreover, in order to remove the bias by the conditions of DNA transfection, the degree of promoter activation may calculate from the normalized enzyme activity ratio by using internal standard enzyme.

It is worried that a compound reduces activity of the internal enzyme extremely has the toxicity over a host cell. So, it is preferable to choose the compound, which reduces activity of the index enzyme in the downstream of the promoter without reducing the activity of the internal enzyme.

### The Best Mode for the Invention

Although a case of the example is given to below and this invention is explained more concretely, these do not restrict the range of this invention.

### Example 1: Cloning of rat GAPDH gene promoter domain

It carried out using the promoter finder DNA walking kit (Clontech) of the rat genome DNA origin. The library, which contains genome DNA fragments, given by either EcoRV or DraI digestion was used.

In this library, since the following adapters (SEQ ID NO: 7) are added to 5' end, PCR is possible by using following primer (adapter primer (AP1) and then nested adapter primer (AP2)). adapter;

5'-GTAATACGAC TCACTATAGG GCACGCGTGG T-3' (SEQ ID NO: 7)
AP1;

5'-GTAATACGAC TCACTATAGG GC-3' (SEQ ID NO: 8)
AP2;

5'-ACTATAGGGC ACGCGTGGT-3' (SEQ ID NO: 9)

Anti-sense primer 1(GAP1) and nested anti-sense primer 2 (GAP2) were synthesized chemically, using the sequence near start codon of 5'-end cDNA of rat GAPDH (GenBank registration No. AB017801).

(The numbers with underline show nucleic acid sequence number in rat GAPDH A of GenBank registration No. M17701.)
GAP1; 
197 5'-CCATTGAACT TGCCGTGGGT AGAATCAT-3' 170 (SEQ ID NO:
10)
 GAP2;

120 5'-GGCAACAATG TCCACTTTGT CACAAGAG-3' 93 (SEQ ID NO:
11)

In order to isolate a promoter, nested PCR using two sorts of following DNA polymerase was performed to the two above-mentioned libraries, respectively.
(1) Advantage Tth polymerase mix (#8418-1; Clontech)
   It prepared and used so that it might be set to 5-6 unit / 50 micro L per reaction solution.
(2) Taq Plus Long polymerase mixture (#600203; Stratagen)

It prepared so that it might be set to 5 units / 50 micro L per reaction solution, and to the solution 1.1 micro L/50 micro L of the Taq start antibody (#54001; Clontech) was added.

Continuation nested PCR using GAP1 and GAP2 performed PCR using the GeneAmp PCR system 2400 (Perkin-Elmer).

### Primary reaction: using primers AP1 and GAP1,

94 degrees C, 2 seconds,
71 degrees C, 3 minutes x 7 cycles
94 degrees C, 2 seconds,
66 degrees C, 3 minutes x 32 cycles
66 degrees C, 4 minutes.
Secondary reaction: using primers AP2 and GAP2,
94 degrees C, 2 seconds,.
73 degrees C, 3 minutes x 5 cycles
94 degrees C, 2 seconds,
68 degrees C, 3 minutes x 20 cycles
68 degrees C, 4 minutes.

The 5.3kb unique amplified fragment was obtained by PCR from the DraI library, and it contained the restriction enzyme recognition sites of EcoRV (2.5kb), ScaI (1.9kb), and PvuII (0.7kb).

By EcoRV digestion, the 2.5kb fragment was excised and it was named the promoter 302 (p302).

On the other hand, since the 1.2kb unique amplified fragment was obtained from the EcoRV library and the restriction enzyme map between above-mentioned p302 and the 1.2 kb fragment were different from each other, so the 1.2 kb fragment was named the promoter 104 (p104).

P302 and p104 obtained were inserted in the SrfI site of the plasmid (pCR-Script Amp SK(+); Stratagen) respectively, E. coli (XL1-Blue MRF'; Stratagen) was transformed by the plasmid and cultured.

Positive colony was chosen by colony-hybridization with synthetic rat 5'-end cDNA of GAPDH nucleotide as a probe: 
12 5'-CGGTGTGAAC GGATTTGGCC GTATCGGA-3' 39(SEQ ID NO:
12)
 
30 5'-CCGTATCGGA CGCCTGCTTA CCAGGG-3' 55 (SEQ ID NO:
13)
 (The numbers with underline show nucleic acid sequence number in rat GAPDH which set the point starting to 1 (ATG translation point).)

The plasmid extracted from the positive colony was purified and the nucleic acid sequences of p104 and p302 were determined by the usual method. Each sequences are shown in the SEQ ID NO: 1 (p104) and the SEQ ID NO: 2 (p302).

### Example 2: Identification of the GAPDH gene promoter that induces cell death

The following experiments were conducted in order to determine the promoter portion that induces cell death process from a fragment including the promoter domain obtained in the example 1.

The plasmid which inserted promoter sequence (p104-pCR-Script Amp SK (+) and p302-pCR-Script Amp SK (+)) were cleaved at BsmBI site which exists just upstream of translation starting site of GAPDH protein and was created blunt-ends. The fragment was cleaved at the MluI site of 5'-end, sequence including promoter sequence was excised.

The pGL3-enhanser vector (Promega) was opened the M1uI-SmaI site, and each promoter sequence excised here was inserted.

Reporter assay was performed with each vector as follows. Reporter assay used the primary culture cell of rat cerebellum origin granule cells (CGCs).

CGCs were prepared according to the previous report (J. Neurochem., 66,928-935 (1996)). 16 hours after plating, the following were mixed and added to the non-serum cultures. In addition, it mixed beforehand and added in the culture so that the ratio of A and B might be set to 5:1, it might serve at a final concentration of 2 micro g/mL, C might be set to 1 micro L/micro g **DNA** and D might be set to 6 micro L/micro g **DNA.**
A) p104-pGL3-enhanser vector or p302-pGL3-enhanser vector both vector contains a firefly luciferase gene on the downstream of the promoter.
B) pRL-SV40 vector (it includes Renilla luciferase gene as an internal standard enzyme on the downstream of SV40 promoter currently activated constantly),
C) LIPOFECTAMINE (GIBCO BRL)
D) LIPOFECTAMINE PLUS Reagent (GIBCO BRL)

The cells were washed 20 hours after plating and the culture was continued for further 4 hours. Next, cytosine arabinoside (cell death inducing agent; AraC) was added at a final concentration of 500 micro M and then cells were cultured for 14 hours or 24 hours.

The probability of survival of CGCs treated with AraC (500 micro M) was 68% for 14 hours and 44% for 24 hours. On the other hand, CGCs treated with AraC (10 micro M) was more than 90% of probability of survival, and significant cell death did not occurred at 14 or 24-hours culture (control).

The cells cultured with AraC (500 micro M) for 14 hours and 24 hours were lyzed, and luciferase activity was measured. A result is shown in Table 1 and 2 (luciferase activity of the cell which introduced in the pGL3-enhanser vector that does not contain a promoter was set to 1.0.).

**Table 1**

| DNA introduced | Culture time after addition 14 hours | Culture time after addition 24 hours |
|---|---|---|
| blank(control) | 1.0 | 1.0 |
| p302 | 0.6 | 0.6 |
| p104 | 1.7 | 2.4 |

**Table 2**

| DNA introduced | Culture time after addition 14 hours | Culture time after addition 24 hours |
|---|---|---|
| blank(control) | 1.0 | 1.0 |
| p302 | 0.4 | 0.5 |
| p104 | 1.2 | 1.3 |

The promoter (p104) was activated in a culture time dependent manner, reaching to the 1.7 times increase of luciferase activity in 24 hours after AraC addition in the CGCs group transformed by p104-pGL3-enhanser vector.

The promoter's (p302) activation was not accepted but showed a tendency to decrease rather in the CGCs group transformed by p302-pGL3-enhanser vector (Table 1).

With AraC (10 micro M) disposal, significant cell death could not be induced but neither of the two promoters (p104 and p302) showed significant activation on that occasion (Table 2).

This showed that the sequence shown by p104 had the promoter activity, which induces cell death process.

### Example 3: Screening for compound which suppresses pro-apoptotic GAPDH promoter activity

Plasmid (p104-pGL3-enhanser vector) having promoter DNA was added to the CGCs culture and transformation was carried out, as performed in the example 2.

The cells were washed 20 hours after plating and cultivation continued for further 4 hours. Next, AraC (cell death inducing agent) was added at a final concentration of 500 micro M and cultured for 14 hours or 24 hours.

In addition, 1 hour before AraC exposure, tested compound was added so that it might become at a final concentration of 0.001 - 10 micro M.

The cell was lyzed after culturing, luciferase activity was measured, and the inhibition rate of GAPDH promoter activity with the tested compound was calculated.

The inhibition rate made 0 % in the case where only a solvent was added, and the inhibition rate made 100 % in the case without the rise of significant Firefly luciferase activity.

## Claims

1. An isolated and purified rat glyceraldehyde-3-phosphate dehydrogenase (GAPDH) promoter DNA.

2. A promoter DNA according to claim 1 that consists of the nucleotide sequence shown in SEQ ID NOs: 1, 3, 5 or 6 or a complementary DNA sequence thereof.

3. A vector inserting the DNA according to claim 1 or 2.

4. A host cell transformed with the vector according to claim 3.

5. A screening method for apoptosis suppresser by using the rat promoter DNA according to claim 1.

6. A screening method for apoptosis suppresser by using the vector according to claim 3.

7. A screening method for apoptosis suppresser by using the host cell according to claim 4.

8. A screening method for apoptosis suppresser which comprises:
1) inserting rat GAPDH promoter into a vector,
2) transforming host cells with the vector,
3) adding a tested compound and
4) measuring survival ratio of the host cells.


SEQUENCE LISTING

<110> Ono Pharmaceutical Co., Ltd.

<120> Promoter for glyceraldehyde-3-phosphate dehydrogenase which induces
apoptosis.

<130> 13071/CH/ajk

<140> EP 01955624.0
<141> 2001-08-09

<150> PCT/JP01/06858
<151> 2001-08-09

<150> JP 2000-242014
<151> 2000-08-10

<160> 13

<210> 1
<211> 1124
<212> DNA
<213> Rattus norvegicus

<220>
<221> CDS
<222> (1035)..(1124)

<400> 1
actatagggc acgcgtggtc gatggcttgg gttggtatct acgactaaac aagagagaga 60

atgagattaa tctccgcata atagtgtgta tcggctgaac atacacagac acacacagac 120

acacacatac acacagacac agaaacatac gcttagcaca cacagaagca cacatacaca 180

gacagaaact cacatacaca gagacccaca gatatagaca cacagagaca cagatgcaca 240

cacagaccta cttacacact catagacata gatacacaca catagacaca cactgacata 300

tcaacacacc tacagacaca cacaccacac atacgcacac acacatacat gaacacacac 360

atacacacgc acacacatac acacacatgg aaacacatgt tttatgaaga tctaggaatt 420

accccaatca cagtagaatg gatgcctgtg ttgggaccca ggagggacag gaaatagaaa 480

tgggctaggg ggctggagag atggctcaga ggttaagagc actgtctgct cttccagagg 540

tccggagttc aattcccaga aaccacatgg tggctcacaa ccatctataa tcaggtctgg 600

tgccctcttc tggcctgcag gcatacatgc aagcagaaag ctgtatgatg tatacataat 660

aaataaataa atttgaaaaa aaagaaatgg gcttaggggt gatgggaaga gaaggactct 720

gaaggtctgt agaccgctca taaactgggg cagacgttgc tcctgcttga gcacctgact 780

tcccagtcca ttgccaccat ggtctttgtg ccaccatggc cagtccaagg ccatccttaa 840

acaggctgca aaaagtttta actaggacca ttagattgtc tcttaaaact atgaaaggtt 900

ttactttatt tctctctctc tctctctctc tctctctctc tcattttaaa aatatttttt 960

cggctctctg ctcctccctg ttctagagac agccgcatct tcttgtgcag tgccagcctc 1020

gtctcataga caag atg gtg aag gtc ggt gtg aac gga ttt ggc cgt atc 1070
Met Val Lys Val Gly Val Asn Gly Phe Gly Arg Ile
1               5                   10

gga cgc ctg gtt acc agg gct gcc ttc tct tgt gac aaa gtg gac act 1118
Gly Arg Leu Val Thr Arg Ala Ala Phe Ser Cys Asp Lys Val Asp Thr
        15                  20                  25

gtt gcc 1124
Val Ala
    30

<210> 2
<211> 30
<212> PRT
<213> Rattus norvegicus

<400> 2

Met Val Lys Val Gly Val Asn Gly Phe Gly Arg Ile Gly Arg Leu Val
1               5                   10                  15

Thr Arg Ala Ala Phe Ser Cys Asp Lys Val Asp Thr Val Ala
            20                  25                  30

<210> 3
<211> 2565
<212> DNA
<213> Rattus norvegicus

<220>
<221> CDS
<222> (2476)..(2565)

<400> 3
actatagggc acgcgtggtc gacggcccgg gctggtaaaa aggaacgttg cagcctgccc 60

agtgaccctc tcccccgccc ctgtggtttc cattcttaag tgtttatctt ccatgctggc 120

tggcatttta tgatgatcct gataaccaca gcctacgttt agagattaga tgaatccggc 180

tctcgtttca acaaagaaat gggttttccg attaagctct gctctgagga catgaaaaat 240

ccaatggtcc cctccttctc atgcaattgt catacaacct cctgtctgta atacaaacaa 300

actcttattc aggtataatt tatgatgcct gtcgtctttg tgtttccctt ttcagtttta 360

ttaaattgcc attagccccg tcgtccgtcc tctctacttt taccctgtgg ttttctacat 420

ctcttgttga atttgtctta aaaatgtgtt cctattgcta agaagctcca gcatggagta 480

cctggttcta cacctagaca aggatgcaga gtaaaagttg cagccccact ggcacaggta 540

tttcaaacca aacagcagct gaccctgtga ttcctccagt catgcctggg ccataatgta 600

aagtctctct tgctacaagg aatcctaaag ctacatatat atatagtgtc tgtgtctgct 660

tggagtgcaa aagaccctga acaatgaggt cactgtcaca ctgaaccagt acagtgtcat 720

aggaattctg agccccatag taagacagaa gtgtagtgcc attagtgtca aaagaaacat 780

gaatatggga ggttatcaga ctagaatagc ttcaaacact cttacaggaa ccacacagta 840

cttgatcact tgtaactgaa agattggtat gaaggcccag aaagctcaaa agatatattc 900

tctctctccc tctccttctt cccctccacc tttctctccc ccttcccctc cccccttctc 960

tctctgggac agcatcttac tatgtagccg tgttgatctg aaactcacta tagaaaacgg 1020

gctggtcctg actcatgctg ctgccccact tctgcttcgt gagtgatgga gttaaagaac 1080

tacaccactg cacctcactc catctatctg tctgtgtctg tctctctctc tctctctttc 1140

ttactaacac ctccgctcac attctgtttg cgaggatgag tctttgccac acagaattca 1200

atggatacaa atgtacatct ttaggccccc attaaattgt cacggtcagg ccttgccccc 1260

tttagtcaac tgctcctgga agaagtcaca gcagacatgc ctctacctgc tgcacagccg 1320

ctcggattgg cgggggacag accctaccaa tggaggtaca ctgtgcagtc actgtgccaa 1380

gagaggccct cactcggaaa cacatgcccc attctttctc atgtaatgct ctcttctcag 1440

tttagagtga gaacatctgg caaggatgaa gatgggcgat ggtgtcatct agacctccca 1500

aaagatgacg tcactgaacc cggtgaaaga acccacagcg gaagccccaa aggctgacgc 1560

cctaatctct tagcaaatgg aacaagataa tatgacaccg tgccaagatg aaattggctg 1620

tcccatggag agcactatgc tttaagtcat gaaaccaatg gccaaagtga tatgcaacaa 1680

gaattaggta taagttttgt acacacacac ccgcgcacac acacacgcac atatgcacat 1740

gcacacgcac acccatgcac acatgcatgc acacttttcc ttaaactcaa cattttttcc 1800

tattagaagt tatacctgaa aaagactggc atactgtata tattcaaatg acttttaata 1860

tacacactac acacttgcat tcagatgcat gctgggagca aacagcccag acaagccttg 1920

cgaaaacatc aagagtcttc ccggtgcgca tgagttcttt cagcgggtgt tgtttgtgtc 1980

ctcagaacat tcagcacgca ctgctaacac accaaaccac tcctgtagag agtacagcac 2040

atttgaaaag tgaaaatctt aaagaagttc ttcactaaaa gttagatggg atttttgttt 2100

tctatttatg ccaaggagtc ctttgcgtgc gtgtgtgtct gtgcaccgtg tgtgtacccc 2160

tgcccaggga agccagaaga gggcatcaga tcccccagga actggagtta tgggtagttg 2220

taagttacca ctcggtacct gagatcaaac ccaggtcctc tgggatagcc agtgctctta 2280

aacactgagc catcatcatc tccgctgaga tttttagact cagttccaac ccatctcctg 2340

tctttttcca aggaagaggg ggtattttgc agaaatcaat gaaggcaaag accagaaagg 2400

taggctctct gctcctccct gctccagaga cagccgcacc ttctcgtgca gtgccagcct 2460

cgtctcatag acaac atg gtg aag gtc ggt gtg aac gga ttt ggc cat atc 2511
Met Val Lys Val Gly Val Asn Gly Phe Gly His Ile
1               5                   10

ggg cgc ctg gtt acc agg gct gcc ttc tct tgt gac aaa gtg gac att 2559
Gly Arg Leu Val Thr Arg Ala Ala Phe Ser Cys Asp Lys Val Asp Ile
        15                  20                  25

gtt gcc 2565
Val Ala
    30

<210> 4
<211> 30
<212> PRT
<213> Rattus norvegicus

<400> 4
Met Val Lys Val Gly Val Asn Gly Phe Gly His Ile Gly Arg Leu Val
1               5                   10                  15

Thr Arg Ala Ala Phe Ser Cys Asp Lys Val Asp Ile Val Ala
            20                  25                  30

<210> 5
<211> 1034
<212> DNA
<213> Rattus norvegicus

<400> 5
actatagggc acgcgtggtc gatggcttgg gttggtatct acgactaaac aagagagaga 60
atgagattaa tctccgcata atagtgtgta tcggctgaac atacacagac acacacagac 120
acacacatac acacagacac agaaacatac gcttagcaca cacagaagca cacatacaca 180
gacagaaact cacatacaca gagacccaca gatatagaca cacagagaca cagatgcaca 240
cacagaccta cttacacact catagacata gatacacaca catagacaca cactgacata 300
tcaacacacc tacagacaca cacaccacac atacgcacac acacatacat gaacacacac 360
atacacacgc acacacatac acacacatgg aaacacatgt tttatgaaga tctaggaatt 420
accccaatca cagtagaatg gatgcctgtg ttgggaccca ggagggacag gaaatagaaa 480
tgggctaggg ggctggagag atggctcaga ggttaagagc actgtctgct cttccagagg 540
tccggagttc aattcccaga aaccacatgg tggctcacaa ccatctataa tcaggtctgg 600
tgccctcttc tggcctgcag gcatacatgc aagcagaaag ctgtatgatg tatacataat 660
aaataaataa atttgaaaaa aaagaaatgg gcttaggggt gatgggaaga gaaggactct 720
gaaggtctgt agaccgctca taaactgggg cagacgttgc tcctgcttga gcacctgact 780
tcccagtcca ttgccaccat ggtctttgtg ccaccatggc cagtccaagg ccatccttaa 840
acaggctgca aaaagtttta actaggacca ttagattgtc tcttaaaact atgaaaggtt 900
ttactttatt tctctctctc tctctctctc tctctctctc tcattttaaa aatatttttt 960
cggctctctg ctcctccctg ttctagagac agccgcatct tcttgtgcag tgccagcctc 1020
gtctcataga caag 1034

<210> 6
<211> 2475
<212> DNA
<213> Rattus norvegicus

<400> 6
actatagggc acgcgtggtc gacggcccgg gctggtaaaa aggaacgttg cagcctgccc 60
agtgaccctc tcccccgccc ctgtggtttc cattcttaag tgtttatctt ccatgctggc 120
tggcatttta tgatgatcct gataaccaca gcctacgttt agagattaga tgaatccggc 180
tctcgtttca acaaagaaat gggttttccg attaagctct gctctgagga catgaaaaat 240
ccaatggtcc cctccttctc atgcaattgt catacaacct cctgtctgta atacaaacaa 300
actcttattc aggtataatt tatgatgcct gtcgtctttg tgtttccctt ttcagtttta 360
ttaaattgcc attagccccg tcgtccgtcc tctctacttt taccctgtgg ttttctacat 420
ctcttgttga atttgtctta aaaatgtgtt cctattgcta agaagctcca gcatggagta 480
cctggttcta cacctagaca aggatgcaga gtaaaagttg cagccccact ggcacaggta 540
tttcaaacca aacagcagct gaccctgtga ttcctccagt catgcctggg ccataatgta 600

aagtctctct tgctacaagg aatcctaaag ctacatatat atatagtgtc tgtgtctgct 660
tggagtgcaa aagaccctga acaatgaggt cactgtcaca ctgaaccagt acagtgtcat 720
aggaattctg agccccatag taagacagaa gtgtagtgcc attagtgtca aaagaaacat 780
gaatatggga ggttatcaga ctagaatagc ttcaaacact cttacaggaa ccacacagta 840
cttgatcact tgtaactgaa agattggtat gaaggcccag aaagctcaaa agatatattc 900
tctctctccc tctccttctt cccctccacc tttctctccc ccttcccctc cccccttctc 960
tctctgggac agcatcttac tatgtagccg tgttgatctg aaactcacta tagaaaacgg 1020
gctggtcctg actcatgctg ctgccccact tctgcttcgt gagtgatgga gttaaagaac 1080
tacaccactg cacctcactc catctatctg tctgtgtctg tctctctctc tctctctttc 1140
ttactaacac ctccgctcac attctgtttg cgaggatgag tctttgccac acagaattca 1200
atggatacaa atgtacatct ttaggccccc attaaattgt cacggtcagg ccttgccccc 1260
tttagtcaac tgctcctgga agaagtcaca gcagacatgc ctctacctgc tgcacagccg 1320
ctcggattgg cgggggacag accctaccaa tggaggtaca ctgtgcagtc actgtgccaa 1380
gagaggccct cactcggaaa cacatgcccc attctttctc atgtaatgct ctcttctcag 1440
tttagagtga gaacatctgg caaggatgaa gatgggcgat ggtgtcatct agacctccca 1500
aaagatgacg tcactgaacc cggtgaaaga acccacagcg gaagccccaa aggctgacgc 1560
cctaatctct tagcaaatgg aacaagataa tatgacaccg tgccaagatg aaattggctg 1620
tcccatggag agcactatgc tttaagtcat gaaaccaatg gccaaagtga tatgcaacaa 1680
gaattaggta taagttttgt acacacacac ccgcgcacac acacacgcac atatgcacat 1740
gcacacgcac acccatgcac acatgcatgc acacttttcc ttaaactcaa cattttttcc 1800
tattagaagt tatacctgaa aaagactggc atactgtata tattcaaatg acttttaata 1860
tacacactac acacttgcat tcagatgcat gctgggagca aacagcccag acaagccttg 1920
cgaaaacatc aagagtcttc ccggtgcgca tgagttcttt cagcgggtgt tgtttgtgtc 1980
ctcagaacat tcagcacgca ctgctaacac accaaaccac tcctgtagag agtacagcac 2040
atttgaaaag tgaaaatctt aaagaagttc ttcactaaaa gttagatggg atttttgttt 2100
tctatttatg ccaaggagtc ctttgcgtgc gtgtgtgtct gtgcaccgtg tgtgtacccc 2160
tgcccaggga agccagaaga gggcatcaga tcccccagga actggagtta tgggtagttg 2220
taagttacca ctcggtacct gagatcaaac ccaggtcctc tgggatagcc agtgctctta 2280
aacactgagc catcatcatc tccgctgaga tttttagact cagttccaac ccatctcctg 2340
tctttttcca aggaagaggg ggtattttgc agaaatcaat gaaggcaaag accagaaagg 2400
taggctctct gctcctccct gctccagaga cagccgcacc ttctcgtgca gtgccagcct 2460
cgtctcatag acaac 2475

<210> 7
<211> 31
<212> DNA
<213> Artificial Sequence

<220>
<223> Description of Artificial Sequence:adapter

<400> 7
gtaatacgac tcactatagg gcacgcgtgg t                                    31

<210> 8
<211> 22
<212> DNA
<213> Artificial Sequence

<220>
<223> Description of Artificial Sequence:primer

<400> 8
gtaatacgac tcactatagg gc                                              22

<210> 9
<211> 19

<212> DNA

<213> Artificial Sequence

<220>
<223> Description of Artificial Sequence:primer

<400> 9
actatagggc acgcgtggt                                                  19

<210> 10
<211> 28
<212> DNA
<213> Artificial Sequence

<220>
<223> Description of Artificial Sequence:primer

<400> 10
ccattgaact tgccgtgggt agaatcat                                        28

<210> 11
<211> 28
<212> DNA
<213> Artificial Sequence

<220>
<223> Description of Artificial Sequence:primer

<400> 11
ggcaacaatg tccactttgt cacaagag                                          28

<210> 12
<211> 28

<212> DNA
<213> Artificial Sequence

<220>
<223> Description of Artificial Sequence:probe

<400> 12
cggtgtgaac ggatttggcc gtatcgga                                          28

<210> 13
<211> 26
<212> DNA
<213> Artificial Sequence

<220>
<223> Description of Artificial Sequence:probe

<400> 13
ccgtatcgga cgcctgctta ccaggg                                            26